# EUROPEAN PATENT APPLICATION

(11) **EP 4 492 394 A1**
(43) Date of publication of application: **15.01.2025**
(21) Application number: 23184789.8
(22) Date of filing: 11.07.2023
(51) Int. Cl.: G16H 20/40, A61B 34/10, A61C 13/00, G16H 30/40, G16H 40/63, G06T 19/00, G16H 50/50

(54) **A SYSTEM AND METHOD FOR PLANNING A DENTAL PROCEDURE USING AUGMENTED OR VIRTUAL REALITY**

(71) Applicant: Nulty, Adam Brian, Bury, Lancashire BL9 0NT (GB); Zachrisson, Patrik Magnus Fredrik, Bury Lancashire BL9 0NT (GB)
(72) Inventor: Nulty, Adam Brian, Bury, Lancashire BL9 0NT (GB); Zachrisson, Patrik Magnus Fredrik, Bury Lancashire BL9 0NT (GB)
(74) Representative: Doherty, William

(57) **Abstract**

A system (10) is provided for planning a dental procedure using augmented or virtual reality which comprises a scanning means (12) for creating a digital representation of a head or face of a patient, with at least one scan flag associated therewith, a video capture device (16) for receiving a video input (24) of a dental surgical site in real time, an augmented reality or virtual reality display device (18), and a processor (20) associated with the augmented reality or virtual reality display device (18). The processor (20) is configured to determine an augmented reality or virtual reality overlay based on the digital representation, and display the augmented reality or virtual reality overlay on the augmented reality or virtual reality display device (18) in real-time by matching the at least one scan flag to a corresponding feature of the video input (24), wherein the augmented reality or virtual reality overlay comprises a dental-procedure-planning overlay component (32a, 32b, 32c, 32d) indicative of a dental procedure to be performed.

## Description

The present invention relates to a system for planning a dental procedure using augmented or virtual reality. The invention further relates to a method of planning a dental procedure using augmented or virtual reality utilising said system.

It is common during dental procedures such as dental implant surgery to scan the face of the patient, for example using a CBCT scanning device, to obtain detailed information on the internal structure of the patient's face. The scan data is used in identify how to best conduct dental surgery on the patient.

One issue with current planning is the difficultly in visualising how the patient will look after the procedure. If stock or standard dental components are being used, it is a challenge for the dentist to decide which components would best suit the procedure. Additionally, the dentist and patient cannot easily be shown a representation of the dental surgical site post-procedure, meaning decisions on the dental procedure have to be made in the absence of this information.

Procedures such as dental implant surgery also involve serious risks of damaging vital structures such as nerves or sinus cavities when drilling into the jaw and placing the implant. These vital structures can only be seen on the scan data, so the dentist may have to intuit where these structures are by referring back and forth between the scan data and their view, which is a difficult process and increases the probability of errors.

It is an object of the present invention to reduce or substantially obviate the aforementioned problems, by providing a system and method for planning a dental procedure that reduces the demands on dental practitioners, minimise the risks of accidental damage to the patient, and increase the efficiency of dental planning.

According to a first aspect of the invention there is provided a system for planning a dental procedure using augmented or virtual reality, the system comprising: a scanning means for creating a digital representation of a head or face of a patient, the digital representation comprising at least one scan flag associated therewith, a video capture device for receiving a video input of a dental surgical site in real time, an augmented reality or virtual reality display device, and a processor associated with the augmented reality or virtual reality display device, the processor being configured to determine an augmented reality or virtual reality overlay based on the digital representation, and display the augmented reality or virtual reality overlay on the augmented reality or virtual reality display device in real-time by matching the at least one scan flag to a corresponding feature of the video input, wherein the augmented reality or virtual reality overlay comprises a dental-procedure-planning overlay component indicative of a dental procedure to be performed.

A system for planning a dental procedure using virtual or augmented reality can reduce the demands on dental practitioners, minimise the risks of accidental damage to the patient, and increase the efficiency of dental planning. Procedures can be planned more easily and to a higher standard with the aid of an augmented reality or virtual reality overlay which matches the scanning data and video input which are needed in conjunction to plan dental procedures and can display them in superposition. Additionally, a dental-procedure-planning overlay component can provide the dentist with visual cues to aid the procedure, allowing them to move more easily plan the placement and design of dental surgical components such as implants or surgical guides without causing any discomfort to the patient. Furthermore, the system allows for a post-procedure representation of the patient to be viewed, which can better inform the choice of dental procedure.

Preferably, the augmented reality or virtual reality display device may comprise an interactive user interface.

An interactive user interface allows the user to interact with the augmented reality or virtual reality display device, allowing for better visualisation and display of information.

Advantageously, the interactive user interface may comprise a plurality of library dental-procedure-planning overlay components displayable in the augmented reality or virtual reality display device.

In this case, the dentist may choose suitable dental-procedure-planning overlay components from the library dental-procedure-planning overlay components to suit the procedure to be performed.

Optionally, the interactive user interface may allow alteration of the digital representation and/or the dental-procedure-planning overlay component in the augmented reality or virtual reality display device.

Alteration of the digital representation and/or the dental-procedure-planning overlay component via the interactive user interface can allow for better planning of the procedure. For example, the dentist could view alternative angles of the digital representation of the head or face of the patient, highlight relevant sections, or view magnified sections of the digital representation or video input. It is appreciated that this list of examples is non-exhaustive.

Optionally, the scanning means may comprise an X-ray scanning device and/or a CBCT scanning device.

X-ray and CBCT scanning devices are commonly used in dental services and provide a convenient means to scan the internal structure of a patients face.

Optionally, the processor may comprise artificial intelligence or machine learning algorithms capable of automatically identifying assistive dental-procedure-planning overlay component for display in the augmented reality or virtual reality overlay.

Artificial intelligence or machine learning algorithms can facilitate the planning of a dental procedure by automatically identifying assistive dental-procedure-planning overlay components for display that could be useful for the dentist in planning the dental procedure.

Optionally, the system may comprise at least one dental tool having a detectable component identifiable by the processor when in the video input.

Dental tools such as drills or picks are useful and sometimes necessary for carrying out a wide variety of dental procedures. A dental tool being identifiable by the processor allows for information such as the distance from the dental tool to a tooth or nerve to be tracked and potentially displayed in real-time.

Optionally, the dental-procedure-planning overlay component may comprise a representation of a dental implant or restoration.

Dental restoration and dental implant surgery are two common types of dental procedure. Having a representation of dental implants or restoration in the overlay aids the dentist in planning the procedure, as it can give visual cues on positioning and placement. Additionally, it allows the dentist and/or patient to view a post-procedure model representation of the patient.

Preferably, the dental-procedure-planning overlay component may be manipulable or modifiable in the interactive user interface.

Manipulation or modification of the dental-procedure-planning overlay component allows for the dentist to reshape and resize dental-procedure-planning overlay components to accommodate for the specific facial structure of the patient. This has obvious cosmetic benefits, but could also for instance be used to modify the size of a dental implant to prevent skewering a nerve or sinus.

Preferably, a representation of the dental-procedure-planning overlay component is exportable following manipulation or modification for subsequent manufacture.

Having a representation of the dental-procedure-planning overlay component be exportable for subsequent manufacture provides an easy means to create custom components such as crowns or implants to better suit the facial structure of the patient.

Optionally, the dental-procedure-planning overlay component may comprise a pre-determined representation of a tooth or teeth of a patient.

A pre-determined representation of a tooth or teeth displayable on the dental-planning-procedure overlay component has obvious utility in producing post-procedure representations of the patient, which can help inform the choice of dental procedure.

Optionally, the scanning means may be configured to create a plurality of different digital representations of the head or face of the patient, the different digital representations having different facial expressions.

The scanning means being configured in this way allows for the dentist and/or patient to view how the patient will look post-procedure with a variety of different facial representations.

Advantageously, the processor automatically adds additional scan flags to the digital representation based on identified features of the video input of a dental surgical site.

Adding scan flags to the digital representation based on the features of the video input allows the processor to keep track of features of the video input that were not initially present in the digital representation.

Optionally, the digital representation may be displayable in real-time in the augmented reality or virtual reality display device.

Displaying the digital representation on the augmented reality or virtual reality display device allows the dentist and/or patient to view the digital representation in isolation without the video input, which could be useful in the planning and discussion of a dental procedure.

According to a second aspect of the invention there is provided a method for planning a dental procedure using augmented or virtual reality, the method comprising: scanning a digital representation of a head or face of a patient, the digital representation comprising at least one scan flag associated therewith, receiving a video input of a dental surgical site in real time, determining, using a processor, an augmented reality or virtual reality overlay based on the digital representation; and characterised by displaying the augmented reality or virtual reality overlay on an augmented reality or virtual reality display device in real-time by matching the at least one scan flag to a corresponding feature of the video input; wherein the augmented reality or virtual reality overlay comprises a dental-procedure-planning overlay component indicative of a dental procedure to be performed.

The above-detailed method allows for the improved planning of a dental procedure. By matching scan flags of the digital representation produced through a scan of the patient to features on the video input, an augmented or virtual reality overlay can be generated. This overlay can provide effective aid for the dentist in deciding on and planning a dental procedure.

For a better understanding of the present invention, and to show more clearly how it may be carried into effect, reference will now be made by way of example only to the accompanying drawings, in which:
Figure 1 shows a pictorial representation of one embodiment of a system for planning a dental procedure in accordance with the first aspect of the invention;
Figure 2 shows a pictorial representation of a video input of a patient's mouth having an augmented reality or virtual reality overlay displayed on an augmented reality or virtual reality display device with scan flags corresponding to features of the video input, as used in accordance with the system of Figure 1;
Figure 3 show a pictorial representation of an interactive user interface of the system of Figure 1, showing a plurality of library dental-procedure-planning overlay components displayed in the augmented reality or virtual reality device;
Figure 4(a) shows a pictorial representation of the video input of the patient's mouth of Figure 2 having a modified augmented reality or virtual reality overlay and including a plurality of dental-procedure-planning overlay components;
Figure 4(b) shows a pictorial representation of a dental-procedure-planning overlay component of Figure 4(a) in the process of being resized in the modified augmented reality or virtual reality overlay;
Figure 5 shows the modified augmented reality or virtual reality overlay of Figure 4(a), wherein the dental-procedure-planning overlay components have been manipulated in the interactive user interface for subsequent export and manufacture of physical dental implants; and
Figure 6 shows the modified augmented reality or virtual reality overlay of Figure 2, with a dental tool being visible in the video input, and a warning message being displayed in the modified augmented reality or virtual reality overlay;

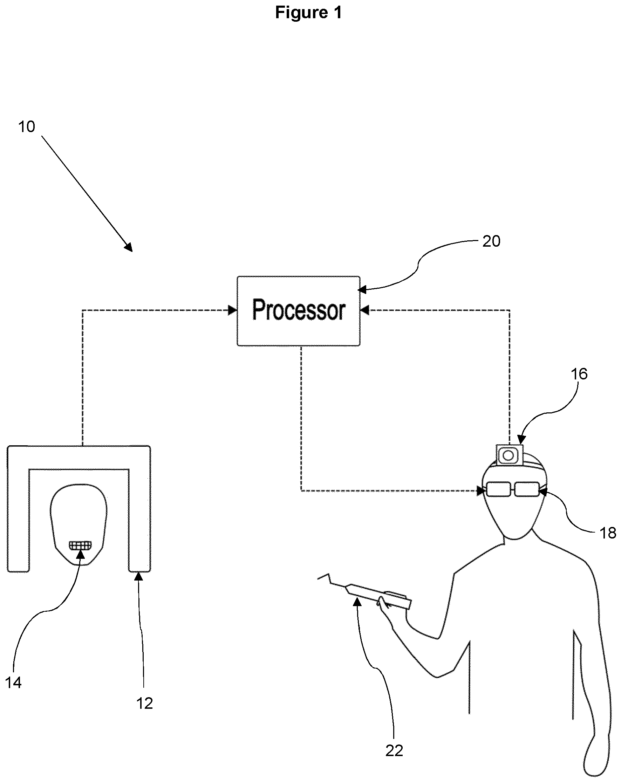

With reference to Figure 1, there is shown a system for planning a dental procedure referenced globally at 10. The system 10 could be used be utilised, for instance, in planning restorative design such as crowns, veneers, inlays and overlays, and bridges, used in planning dental implant surgery, or used as an aid to regular dental check-ups. It is appreciated that this list is non-exhaustive.

The system 10 comprises a scanning means 12 for scanning at least a patient's oral region 14, a video capture device 16, an augmented or virtual reality display device 18, and a processor 20. The system 10 may also comprise a dental tool 22, to be used for the dental procedure.

The scanning means 12 may for example comprise an X-ray scanning device, preferably a CBCT scanning device. Such devices are already commonly used to provide information on the internal structure of the face or head of a patient. The scanning means 12 may comprise an intraoral scanner.

The video capture device 16 may be a provided as a video camera. The video capture device 16 may be mounted in place to capture a view of the dental surgical site or alternatively mounted to the dentist, preferably near eye level, to provide a video input similar to the ocular view of the dentist. The video capture device 16 is provided with one or more lenses, which may have fixed focus lengths or be zoom lenses, allowing for a magnified view.

The augmented or virtual reality display device 18 may be provided as a pair of glasses or goggles as depicted or may, to provide a non-exhaustive list of example devices, be a tablet, smartphone, or monitor.

The processor 20 receives data from the scanning means 12 and the video capture device 16, and can output to the augmented reality or virtual reality display device 18. The processor 20 may be part of a computer at the location of the dental procedure to be performed or accessed via cloud computing, which has the advantage of reducing the equipment required onsite.

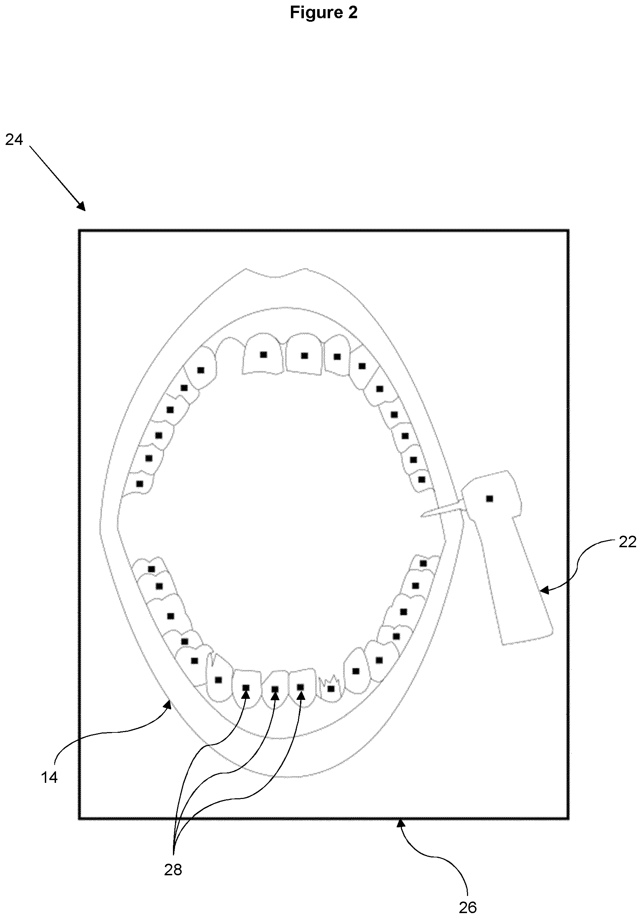

Figure 2 shows a pictorial representation of a video input 24 of the oral region 14 of the patient. An augmented or virtual reality overlay is shown, generated based on scan flags of the digital representation.

The digital representation is created from the scan data obtained by the scanning means 12, and comprises at least one scan flag assigned by the processor 20 to an oral or facial feature of the patient. Scan flags may be stored in point-cloud format, each point in the cloud having three cartesian coordinates identifying its location in three-dimensional space. The digital representation may be obtained by stitching together multiple data sources, such as data obtained from multiple scans.

The processor may automatically identify and assign scan flags to oral or facial features of the patient. The processor may comprise artificial intelligence or machine learning algorithms trained on datasets of facial or oral scan data to identify features such as teeth, cavities, nerves and sinuses. The training data set may be expanded with scans performed by users of the system 10 for planning dental procedures.

Ideally, the digital representation may be viewable in isolation on the augmented reality or virtual reality display device 18. The interactive user interface 26 may allow alteration of the digital representation by the user, for example to rotate the view of the digital representation or to zoom in or highlight specific portions. This alterable view of the digital representation may be helpful for the dentist in planning the procedure, and also provides a visual aid for the dentist to discuss possible dental procedures with the patient.

The overlay depicted in Figure 2 explicitly shows plurality of markers 28, with one for each feature that corresponds to a scan flag of the digital representation. Scan flags may be present for any relevant features of the patient's facial structure such as teeth, tooth cavities, nerves, or sinuses.

To create the display, a preferably injective map between the scan flags and the features of the video input 24 is created by the processor 20. The scan flags are matched to the features of the video input 24 in real time, recalculating as the video input 24 changes. Scan flags may be added automatically to the digital representation by the processor 20, by identifying features of the video input 24 and creating a corresponding scan flag for said features.

Any dental tools 22 present within the video input 24 are identifiable by the processor 20, which can allow for the position of the tool to be tracked in relation to any scan flags. The processor 20 may detect a dental tool 22 through its shape, possibly through the use of artificial intelligence or machine learning algorithms, or alternatively the dental tool 22 may have a detectable component such as for example an identifiable bar code or QR code, or an electronic chip within the dental tool 22 in communication with the processor 20, to track the position of the dental tool 22 more accurately.

The user can ideally interact with the display via the interactive user interface 26, for instance to view a magnified view of the feature, which may be accomplished with a zoom lens of the video capture device 16 or by a digital zoom.

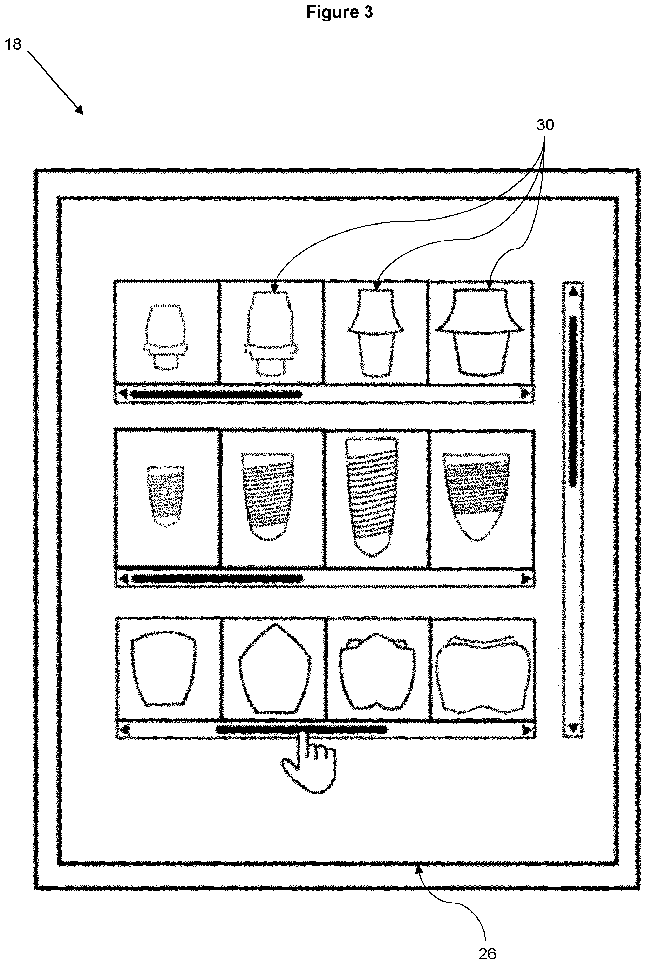

A representation of the plurality of library dental-procedure-planning overlay components 30 displayed in the augmented or virtual reality display device 18 is shown in Figure 3. As illustrated, a user is able to browse through and select one or more library dental-procedure-planning overlay components 30 via the interactive user interface 26. This allows for the procedure to be easily planned by selecting stock components (such as the depicted crowns, abutments, and implants) to fit the procedure.

The library dental-procedure-planning components 30 could include many different components used in dentistry, including prostheses, including but not limited to crowns, bridges, inlays and overlays, and veneers, and surgical components including but not limited to implants, abutments, and surgical guides, as well as abstract overlay components such as arrows or text which may be useful in guiding a dental procedure. Users of the system 10 could export and import library dental-procedure-planning to a repository which may be accessible online or stored on a computer.

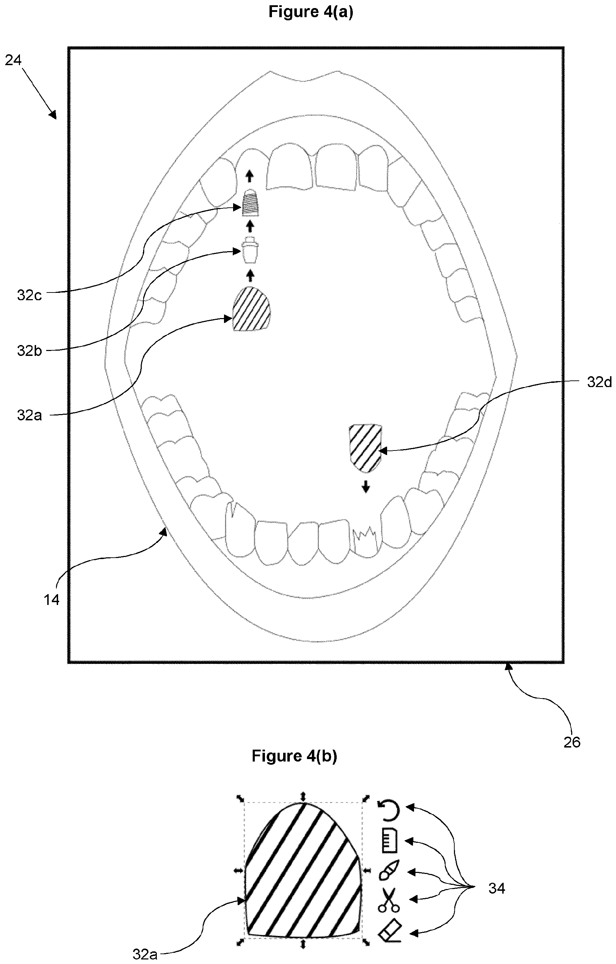

In Figure 4(a) a plurality of dental-procedure-planning overlay components 32a, 32b, 32c, 32d are shown being used to assist in planning a dental procedure on a modified augmented reality or virtual reality overlay, showing two representations of false teeth 32a, 32d, an abutment 32b, and a dental implant 32c. The dental-procedure-planning overlay components 32a, 32b, 32c, 32d may be manually positioned by the user or automatically positioned by the processor 20.

The processor 20 may utilise artificial intelligence or machine learning algorithms to identify assistive dental-procedure-planning overlay components and suggest their placement. The dental procedures carried out by a user of the system 10 may serve as a training data set to improve the suggestions of the processor 20. In the case of the processor 20 being accessed through a cloud computing or data storage environment, the training data set may be generated by a plurality of different users each utilising a system 10 for planning dental procedures in different locations.

The dental-procedure-planning overlay components 32a, 32b, 32c, 32d may be manipulated or modifiable as illustrated in Figure 4(b), which shows the modification of a tooth implant 32a. Through the interactive user interface 26, the user may be able to size, shape, and position the dental-procedure-planning overlay components 32a, 32b, 32c, 32d in three-dimensional space, which could be used, for instance, to achieve the desired bite and occlusion of a crown or bridge. Another example use-case is modifying the size, shape, and position of a dental implant to avoid damaging a nerve or sinus.

The interactive user interface 26 may comprise a plurality of assistive tools 34 to facilitate the modification and manipulation of the dental-procedure-planning overlay components 32a, 32b, 32c, 32d.

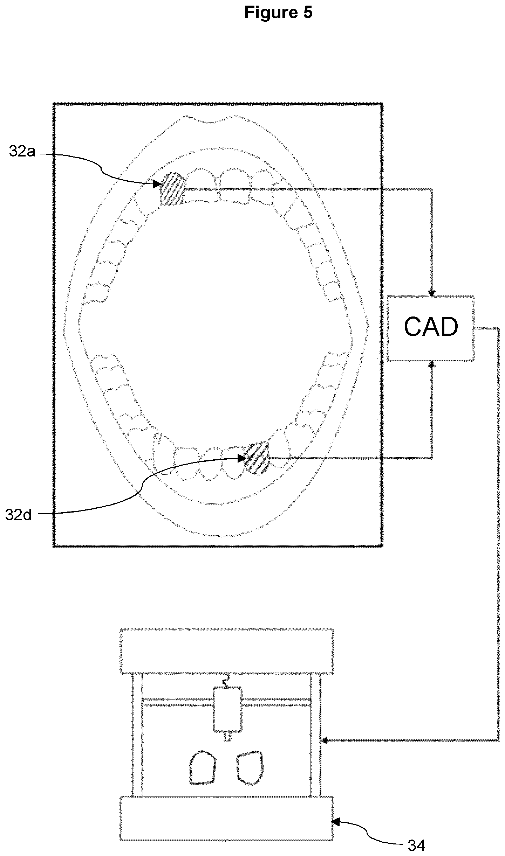

Figure 5 provides a good example of how useful manipulating and modifying the dental-procedure-planning overlay components 32a, 32b, 32c, 32d can be in planning a dental procedure. In this representative illustration, two of the dental-procedure-planning overlay components 32a, 32d shown in Figure 4 have been shaped and sized to fit well between the other teeth of the patient.

The dentist can move the dental-procedure-planning overlay components 32a, 32b, 32c, 32d such as the depicted representation of false teeth into the position the physical false teeth will sit post procedure, to view the resulting appearance of the patient. While the planning of false teeth is shown in Figure 5, the system 10 can evidently be used in the same way to plan crowns, bridges, veneers, inlays and overlays, or any other form of dental prosthetic.

The ease with which the dentist can create post-procedure representations of the patient's appearance means that mock-ups can be quickly created to be shown to the patient for feedback, and amendments can potentially be made. This allows for a prosthesis that the patient approves of to be quickly designed in one sitting, and potentially eliminates the need for manufacturing of prototypes to physically show the user.

Once the patient and dentist have settled on the design of any dental-procedure-planning components, the parameters of the component can be exported for subsequent manufacture by a manufacturing apparatus 34. The manufacturing apparatus 34 could for example comprise a 3D printer, which allows for physical components, including but not limited to false teeth, inlays and overlays, crowns, bridges, and veneers, to be produced on site and almost immediately. Components produced in this way are particularly suitable for temporary prostheses, which are often needed while waiting for harder wearing prostheses to be produced, or while waiting for the patient's jaw to sufficiently recover post-surgery such that permanent prostheses can be installed.

The manufacturing apparatus 34 could comprise a milling machine. This allows for hard-wearing dental prostheses to be precisely milled, and may occur offsite. It is also envisioned that parameters from the dental-procedure-planning components may be exported to the 3D printer, or similar additive manufacturing machine, and a milling machine, to quickly create a temporary prostheses which can later be replaced by a hard-wearing prosthesis produced by the milling machine.

In addition to the prostheses mentioned above, the manufacturing apparatus 34 can also be used to produce other components needed for dental procedures, such as dental implants or surgical guides, allowing for these parts to be specifically designed to suit the patient.

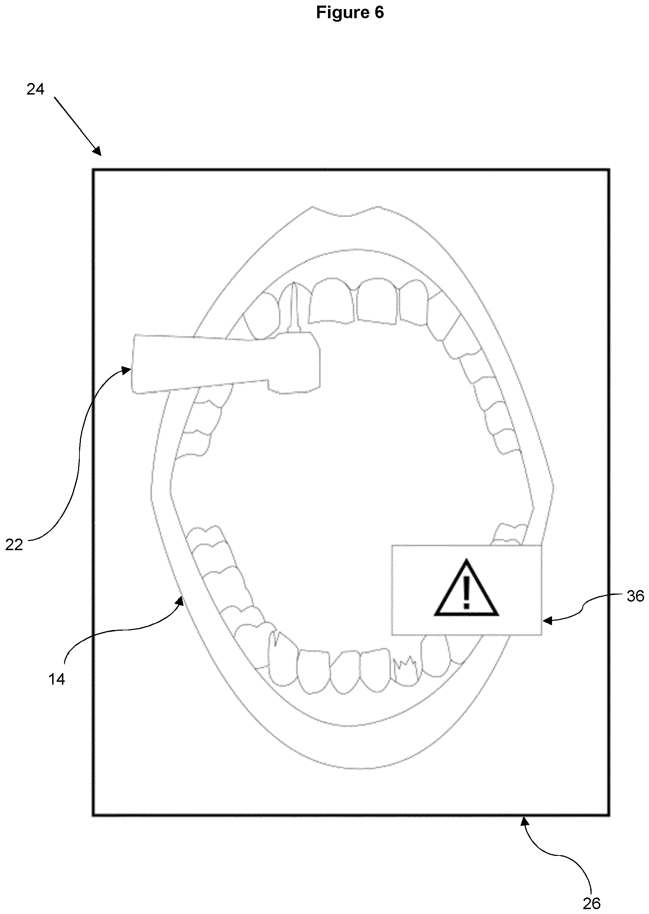

The illustration of Figure 6 illustrates how the system 10 can track and display information that would otherwise be inaccessible to the dentist to facilitate the planning of a dental procedure. The processor 20 can track the position of scan flags and the distances between scan flags, which can be displayed to the dentist to aid in dental procedure planning. This could include information such as the distance between a dental tool vital structures such as nerves or sinuses. One useful possibility of the system 10 is to display a warning 36 if the position of a dental tool is close to a vital structure. Such a warning 36 could prevent accidental damage to a vital structure by the dentist.

To summarise, the present system allows for implementation of a method for planning a dental procedure using augmented or virtual reality, the method comprising: a] scanning a digital representation of a head or face of a patient, the digital representation comprising at least one scan flag associated therewith; b] receiving a video input of a dental surgical site in real time; c] determining, using a processor, an augmented reality or virtual reality overlay based on the digital representation; and d] displaying the augmented reality or virtual reality overlay on an augmented reality or virtual reality display device in real-time by matching the at least one scan flag to a corresponding feature of the video input; wherein the augmented reality or virtual reality overlay comprises a dental-procedure-planning overlay component indicative of a dental procedure to be performed.

It is therefore possible to provide a system for planning a dental procedure using augmented or virtual reality in which a dental surgeon is assisted in the planning steps, by providing an augmented reality overlay which can be used to represent bespoke implant or prosthetic components in real-time, and potentially display them to a patient for selection. This can also lead to rapid manufacture of bespoke implants.

The words 'comprises/comprising' and the words `having/including' when used herein with reference to the present invention are used to specify the presence of stated features, integers, steps, or components, but do not preclude the presence or addition of one or more other features, integers, steps, components, or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of example only, and various changes and modifications will be apparent to persons skilled in the art without departing from the scope of the present invention as defined by the appended claims.

## Claims

1. A system (10) for planning a dental procedure using augmented or virtual reality, the system comprising:
a scanning means (12) for creating a digital representation of a head or face of a patient, the digital representation comprising at least one scan flag associated therewith;
a video capture device (16) for receiving a video input (24) of a dental surgical site in real time;
an augmented reality or virtual reality display device (18); and
**characterised by** a processor (20) associated with the augmented reality or virtual reality display device (18), the processor being configured to determine an augmented reality or virtual reality overlay based on the digital representation, and display the augmented reality or virtual reality overlay on the augmented reality or virtual reality display device (18) in real-time by matching the at least one scan flag to a corresponding feature of the video input (24);
wherein the augmented reality or virtual reality overlay comprises a dental-procedure-planning overlay component (32a, 32b, 32c, 32d) indicative of a dental procedure to be performed.

2. A system (10) as claimed in claim 1, wherein the augmented reality or virtual reality display device (18) comprises an interactive user interface (24).

3. A system (10) as claimed in claim 2, wherein the interactive user interface comprises a plurality of library dental-procedure-planning overlay components displayable in the augmented reality or virtual reality display device (18).

4. A system (10) as claimed in claim 2 or claim 3, wherein the interactive user interface allows alteration of the digital representation and/or the dental-procedure-planning overlay component (32a, 32b, 32c, 32d) in the augmented reality or virtual reality display device (18).

5. A system (10) as claimed in any one of the preceding claims, wherein the scanning means (12) comprises an X-ray scanning device and/or a CBCT scanning device.

6. A system (10) as claimed in any one of the preceding claims, wherein the processor comprises artificial intelligence or machine learning algorithms capable of automatically identifying assistive dental-procedure-planning overlay component (32a, 32b, 32c, 32d) for display in the augmented reality or virtual reality overlay.

7. A system (10) as claimed in any one of the preceding claims, further comprising at least one dental tool (22) having a detectable component identifiable by the processor when in the video input (24).

8. A system (10) as claimed in any one of the preceding claims, wherein the dental-procedure-planning overlay component (32a, 32b, 32c, 32d) comprises a representation of a dental implant or restoration.

9. A system (10) as claimed in claim 8, when dependent on any one of claims 2 to 4, wherein the dental-procedure-planning overlay component (32a, 32b, 32c, 32d) is manipulable or modifiable in the interactive user interface (24).

10. A system (10) as claimed in claim 9, wherein a representation of the dental-procedure-planning overlay component is exportable following manipulation or modification for subsequent manufacture.

11. A system (10) as claimed in any one of the preceding claims, wherein the dental-procedure-planning overlay component (32a, 32b, 32c, 32d) comprises a pre-determined representation of a tooth or teeth of a patient.

12. A system (10) as claimed in any one of the preceding claims, wherein the scanning means (12) is configured to create a plurality of different digital representations of the head or face of the patient, the different digital representations having different facial expressions.

13. A system (10) as claimed in any one of the preceding claims, wherein the processor automatically adds additional scan flags to the digital representation based on identified features of the video input (24) of a dental surgical site.

14. A system (10) as claimed in any one of the preceding claims, wherein the digital representation is displayable in real-time in the augmented reality or virtual reality display device (18).

15. A method for planning a dental procedure using augmented or virtual reality, the method comprising:
a] scanning a digital representation of a head or face of a patient, the digital representation comprising at least one scan flag associated therewith;
b] receiving a video input (24) of a dental surgical site in real time;
c] determining, using a processor (20), an augmented reality or virtual reality overlay based on the digital representation; and **characterised by**
d] displaying the augmented reality or virtual reality overlay on an augmented reality or virtual reality display device (18) in real-time by matching the at least one scan flag to a corresponding feature of the video input (24); wherein the augmented reality or virtual reality overlay comprises a dental-procedure-planning overlay component (32a, 32b, 32c, 32d) indicative of a dental procedure to be performed.
